# EUROPEAN PATENT APPLICATION

(11) **EP 1 439 186 A2**
(43) Date of publication of application: **21.07.2004**
(21) Application number: 04007519.4
(22) Date of filing: 18.12.2000
(51) Int. Cl.: C07H 17/08

(54) **Intermediates for novel antibacterial and prokinetic macrolides**

(30) Priority: 29.12.1999 US 173434 P
(62) Divisional of application: 00311317.2
(71) Applicant: Pfizer Products Inc., Groton, Connecticut 06340 (US)
(72) Inventor: Kaneko, Takushi, Groton, Connecticut 06340 (US); McMillen, William Thomas, Fishers, Indiana 46032 (US)
(74) Representative: Wood, David John

(57) **Abstract**

This invention relates to intermediates that are useful in the preparation of novel erythromycin antibacterial and antiprotozoal agents.

## Description

### Background of the Invention

This invention relates to novel macrolide compounds that are useful as antibacterial, antiprotozoal and/or prokinetic agents in mammals, including man, as well as in fish and birds. This invention also relates to pharmaceutical compositions containing the novel compounds and to methods of treating bacterial and protozoal infections and gastrointestinal disorders in mammals, fish and birds by administering the novel compounds to mammals, fish and birds requiring such treatment.

Macrolides are known to be useful in the treatment of a broad spectrum of bacterial and protozoal infections in mammals, fish and birds. Such antibiotics include various derivatives of erythromycin A such as azithromycin which is commercially available and is referred to in United States patents 4,474,768 and 4,517,359, both of which are incorporated herein by reference in their entirety. Other macrolide antibiotics are disclosed and claimed in PCT international application number PCT/IB98/00741, filed May 15, 1998, which designates the United States, and United States Patent 5,527,780, issued June 18, 1996, each of which are incorporated herein by reference in their entirety. Like azithromycin and other macrolide antibiotics, the novel macrolide compounds of the present invention possess activity against various bacterial and protozoal infections as described below.

Macrolides are also known to be useful as prokinetic agents in the treatment of gastrointestinal disorders. Prokinetic agents (also called motility-enhancing agents) are compounds that induce gastrointestinal smooth muscle contractions, both in vitro and in vivo. These agents include agonists of motilin, a 22-amino-acid polypeptide that causes increased motility of several portions of the gut. For an overview of the clinical development of erythromycin derivatives with prokinetic activity, see R. Faghih et al., Drugs of the Future, 23(8):861-872 (1998).

Defects in the normal gastrointestinal motility pattern can lead to the development of painful, debilitating and chronic disorders. Examples of these disorders include gastroesophageal reflux disease, delayed gastric emptying, diabetic gastroparesis, pediatric gastroparesis, anorexia, gall bladder stasis, postoperative paralytic ileus, systemic sclerosis, chronic constipation (colonic inertia), emesis, gastritis, and intestinal pseudoobstruction. Patients suffering from intestinal pseudoobstruction, for example, suffer from constipation, colicky pain and vomiting. These patients cannot tolerate oral feedings and require total parenteral nutrition. A successful prokinetic agent could alleviate the distress associated with such disorders.

Prokinetic agents can additionally be useful in the placement of diagnostic and therapeutic instruments. For example, prokinetic agents may facilitate the insertion of enteral feeding tubes into the proximal small intestine. In intestinal pseudoobstruction, these agents may assist in the decompression of the upper gastrointestinal tract by nasogastric tubal aspiration. Accordingly, there is a need for the development of an effective prokinetic agent.

### Summary of the Invention

The present invention relates to intermediates of the formula 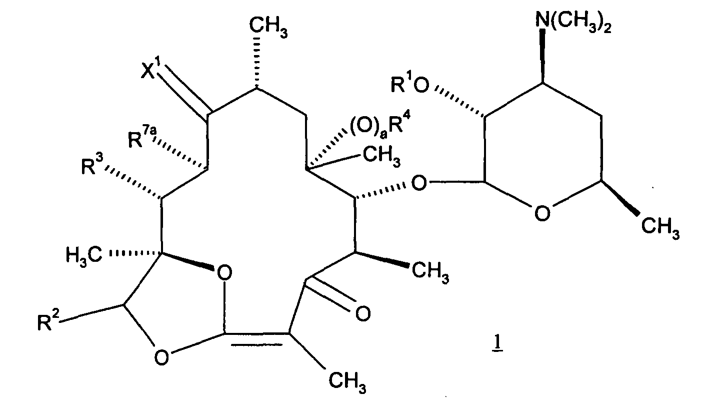 and to salts and solvates thereof, wherein:
a is 0 or 1;
R¹ is hydrogen or a hydroxyl protecting group;
R² is H, C₁-C₆ alkyl, C₃-C₁₀ cycloalkyl, C₅-C₁₀ cycloalkenyl, C₂-C₆ alkenyl, C₂-C₆ alkynyl, (C₁-C₆ alkoxy)C₁-C₆ alkyl, (C₁-C₆ alkylthio)C₁-C₆ alkyl, -(CR¹⁰R¹¹)ₘ(C₆-C₁₀ aryl), and -(CR¹⁰R¹¹)ₘ(4 to 10 membered heterocyclic), wherein each m is independently an integer from 0 to 6, and the foregoing R² groups, except hydrogen, are optionally substituted by one to three R⁸ substituents;
R³ is hydrogen or methyl optionally substituted by one or two groups independently selected from nitro, cyano, R⁶C(O) and R⁶OC(O); or R³ is azido, -OR⁶, -NHR⁶ or -SR⁶;
R⁴ is hydrogen or C₁-C₁₀ alkyl optionally substituted by one or two R⁸ groups;
each R⁶ is independently selected from hydrogen, C₁-C₆ alkyl, C₂-C₁₂ alkenyl, C₂-C₁₂ alkynyl, (C₃-C₁₀ cycloalkyl)C₁-C₆ alkyl, -(CR¹⁰R¹¹)ₘ(C₆-C₁₀ aryl), and -(CR¹⁰R¹¹)ₘ(4 to 10 membered heterocyclic), wherein each m is independently an integer from 0 to 6; and the foregoing R⁶ groups, except hydrogen, are optionally substituted by one to three R⁸ substituents;
R^{7a} is hydrogen or methyl;
X¹ is =O or =NOR⁶;
each R⁸ is independently selected from C₁-C₁₀ alkyl, C₂-C₁₀ alkenyl, C₂-C₁₀ alkynyl, oxo, halo, cyano, nitro, trifluoromethyl, difluoromethoxy, trifluoromethoxy, azido, -OR⁹, -C(O)R⁹, - C(O)OR⁹, -NR¹⁰C(O)OR¹², -OC(O)R⁹, -NR¹⁰SO₂R¹², -SO₂NR⁹R¹⁰, -NR¹⁰C(O)R⁹, -C(O)NR⁹R¹⁰, - NR⁹R¹⁰, -S(O)ⱼ(CR¹⁰R¹¹)ₘ(C₆-C₁₀ aryl), -S(O)ⱼ(C₁-C₆ alkyl), wherein j is an integer from 0 to 2, - (CR¹⁰R¹¹)ₘ(C₆-C₁₀ aryl), -O(CR¹⁰R¹¹)ₘ(C₆-C₁₀ aryl), -NR¹⁰(CR¹⁰R¹¹)ₘ(C₆-C₁₀ aryl), and - (CR¹⁰R¹¹)ₘ(4 to 10 membered heterocyclic), wherein each m is independently an integer from 0 to 4; said alkyl, alkenyl and alkynyl groups optionally contain 1 or 2 hetero moieties selected from O, -S(O)ⱼ- wherein j is an integer from 0 to 2, and -N(R⁹)- with the proviso that two O atoms, two S atoms, or an O and S atom are not attached directly to each other, and the proviso that an O atom, a S atom or a N atom are not attached directly to a triple bond or a non-aromatic double bond; said cycloalkyl, aryl and heterocyclic R² groups are optionally fused to a C₆-C₁₀ aryl group, a C₅-C₈ cycloalkyl group, or a 4 to 10 membered heterocyclic group; and said alkyl, cycloalkyl, aryl and heterocyclic R² groups are optionally substituted by 1 to 5 substituents independently selected from oxo, halo, cyano, nitro, trifluoromethyl, difluoromethoxy, trifluoromethoxy, azido, -NR¹⁰SO₂R¹², -SO₂NR⁹R¹⁰, -C(O)R⁹, -C(O)OR⁹, -OC(O)R⁹, -NR¹⁰C(O)OR¹², -NR¹⁰C(O)R⁹, -C(O)NR⁹R¹⁰, -NR⁹R¹⁰, -OR⁹, C₁-C₁₀ alkyl, -(CR¹⁰R¹¹)ₘ(C₆-C₁₀ aryl), and -(CR¹⁰R¹¹)ₘ(4 to 10 membered heterocyclic), wherein each m is independently an integer ranging from 0 to 4;
each R⁹ is independently selected from H, C₁-C₁₀ alkyl, -(CR¹⁰R¹¹)ₘ(C₆-C₁₀ aryl), and - (CR¹⁰R¹¹)ₘ(4 to 10 membered heterocyclic), wherein each m is independently an integer from 0 to 4; said alkyl group optionally includes 1 or 2 hetero moieties selected from O, -S(O)ⱼ- wherein j is an integer ranging from 0 to 2, and -N(R¹⁰)- with the proviso that two O atoms, two S atoms, or an O and S atom are not attached directly to each other; said cycloalkyl, aryl and heterocyclic R⁹ groups are optionally fused to a C₆-C₁₀ aryl group, a C₅-C₈ cycloalkyl group, or a 4 to 10 membered heterocyclic group; and the foregoing R⁹ substituents, except H, are optionally substituted by 1 to 5 substituents independently selected from oxo, halo, cyano, nitro, trifluoromethyl, difluoromethoxy, trifluoromethoxy, azido, -C(O)R¹⁰, -C(O)OR¹⁰, -OC(O)R¹⁰, - NR¹⁰C(O)R¹¹, -C(O)NR¹⁰R¹¹, -NR¹⁰R¹¹, hydroxy, C₁-C₆ alkyl, and C₁-C₆ alkoxy, and with the proviso that R⁹ must be attached through a carbon atom unless R⁹ is H;
each R¹⁰ and R¹¹ is independently H or C₁-C₆ alkyl;
each R¹² is selected from the substituents provided in the definition of R⁹ except R¹² is not H; and,
with the proviso that when a is zero, R⁴ is hydrogen.

The present invention also relates to compounds of the formula 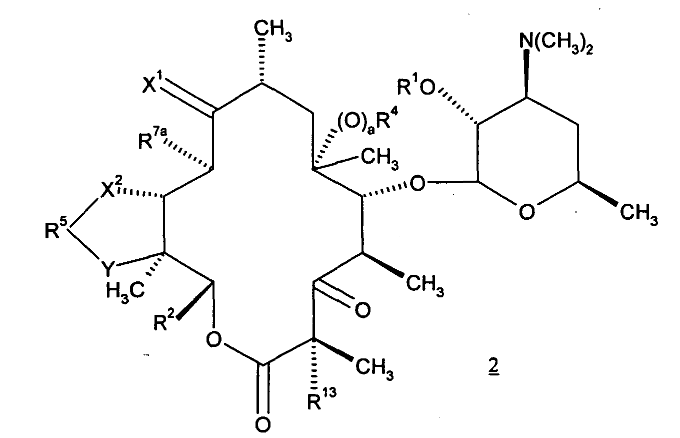 and to pharmaceutically acceptable salts, prodrugs and solvates thereof, wherein:
a, R¹, R², R⁴, R^{7a}, and X¹ are as defined above for said compound of formula 1;
R⁵ is -C(O)-,-C(=NH)-, or (C₂-C₄)alkylene wherein the alkylene group is optionally substituted by one to three R⁸ substituents wherein R⁸ is as defined above for said compound of formula 1;
X² is NH, O, S or CH₂;
Y is NH, O or S;
R¹³ is H or halo.

The present invention also relates to compounds and intermediates of the formula 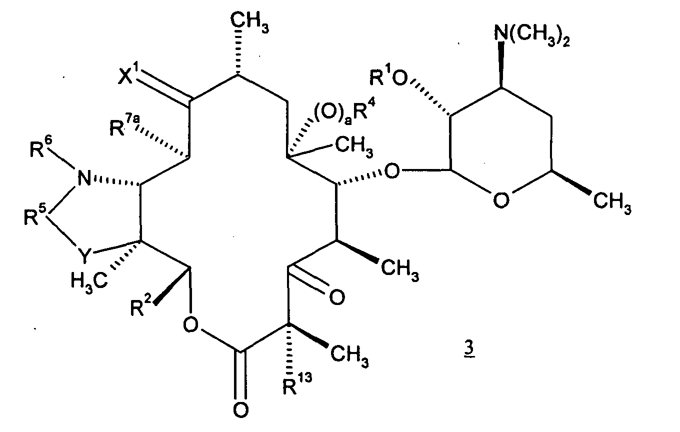 and to pharmaceutically acceptable salts, prodrugs and solvates thereof, wherein:
a, R¹, R², R⁴, R^{7a}, R⁵, R⁶, R¹³, Y and X¹ are as defined above for said compounds of formula 1 and 2.

The present invention also relates to intermediates of the formula 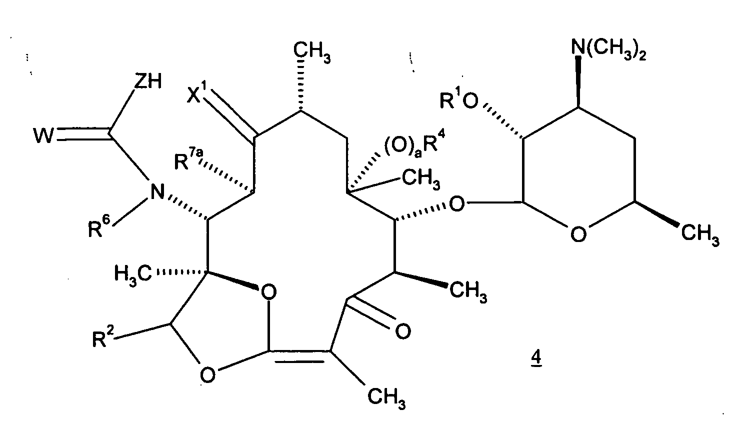 and to salts and solvates thereof, wherein:
a, R¹, R², R⁴, R^{7a}, R⁶, and X¹ are as defined above for said compound of formula 1;
W is O or S;
Z is -N(R⁶)-, O, S, or -CH(R¹⁴) wherein R¹⁴ is H, NO₂, CN, -C(O)R⁹ or C(O)OR⁹; and R⁶ and R⁹ are as defined above for said compound of formula 1.

The present invention also relates to compounds and intermediates of the formula 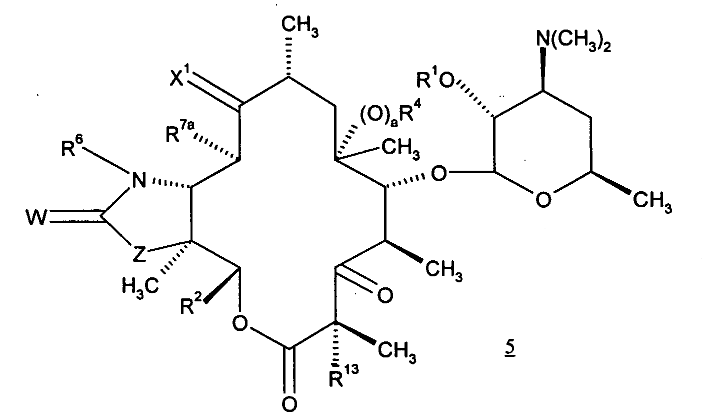 and to pharmaceutically acceptable salts, prodrugs and solvates thereof, wherein:
a, R¹, R², R⁴, R^{7a}, R⁵, R⁶, R¹³, Z, W and X¹ are as defined above for said compounds of formula 1, 2 and 4 but with the proviso that where Z is O then W is not O.

The present invention also relates to intermediates of the formula 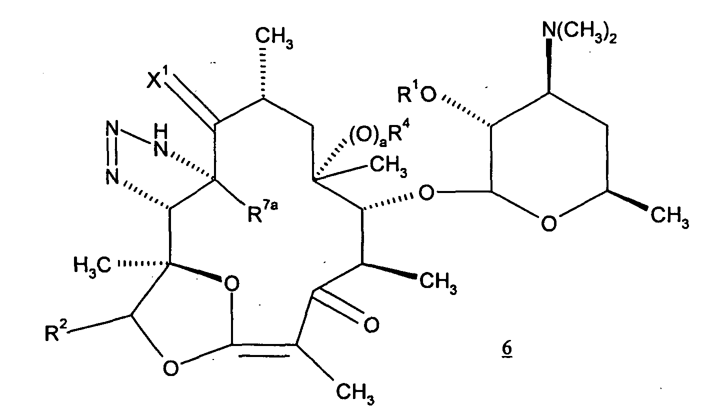 and to salts and solvates thereof, wherein:
a, R¹, R², R⁴, R^{7a}, and X¹ are as defined above for said compound of formula 1.

The present invention also relates to intermediates of the formula 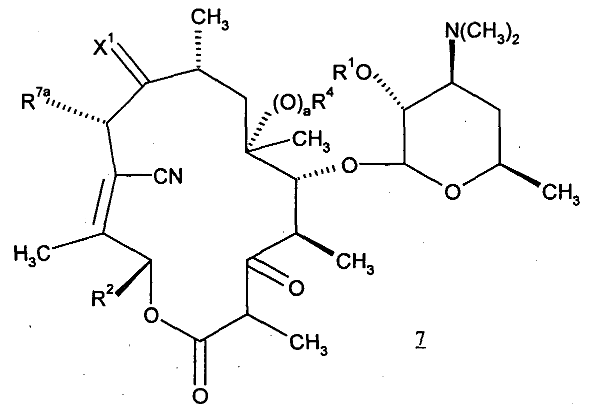 and to salts and solvates thereof, wherein:
a, R¹, R², R⁴, R^{7a}, and X¹ are as defined above for said compound of formula 1.

The invention also relates to a pharmaceutical composition for the treatment of a bacterial infection or a protozoa infection, or a disorder related to a bacterial or protozoal infection, in a mammal, fish, or bird, which comprises a therapeutically effective amount of a compound of formula 2, 3, or 5, or a pharmaceutically acceptable salt, prodrug or solvate thereof, and a pharmaceutically acceptable carrier.

The invention also relates to a method of treating a bacterial infection or a protozoa infection, or a disorder related to a bacterial or protozoal infection, in a mammal, fish, or bird which comprises administering to said mammal, fish or bird a therapeutically effective amount of a compound of formula 2, 3, or 5, or a pharmaceutically acceptable salt, prodrug or solvate thereof.

The invention also relates to a method of treating cancer or atherosclerosis in a mammal which comprises administering to said mammal a therapeutically effective amount of a compound of formula 2, 3, or 5, or a pharmaceutically acceptable salt, prodrug or solvate thereof.

The term "treating", as used herein, unless otherwise indicated, means reversing, alleviating, inhibiting the progress of, or preventing the disorder or condition to which such term applies, or one or more symptoms of such disorder or condition. The term "treatment", as used herein, refers to the act of treating, as "treating" is defined immediately above.

As used herein, unless otherwise indicated, the terms or phrases "bacterial infection(s)", "protozoal infection(s)", and "disorders related to bacterial infections or protozoal infections" include the following: pneumonia, otitis media, sinusitus, bronchitis, tonsillitis, and mastoiditis related to infection by *Streptococcus pneumoniae, Haemophilus influenzae, Moraxella catarrhalis, Staphylococcus aureus, Enterococcus faecalis, E. faecium, E. casselflavus, S. epidermidis, S. haemolyticus,* or *Peptostreptococcus* spp.; pharyngitis, rheumatic fever, and glomerulonephritis related to infection by *Streptococcus pyogenes,* Groups C and G streptococci, *Corynebacterium diphtheriae,* or *Actinobacillus haemolyticum;* respiratory tract infections related to infection by *Mycoplasma pneumoniae, Legionella pneumophila, Streptococcus pneumoniae, Haemophilus influenzae,* or *Chlamydia pneumoniae;* blood and tissue infections, including endocarditis and osteomyelitis, caused by S. *aureus, S. haemolyticus, E. faecalis, E. faecium, E. durans,* including strains resistant to known antibacterials such as, but not limited to, beta-lactams, vancomycin, aminoglycosides, quinolones, chloramphenicol, tetracylines and macrolides; uncomplicated skin and soft tissue infections and abscesses, and puerperal fever related to infection by *Staphylococcus aureus,* coagulase-negative staphylococci (i.e., *S. epidermidis, S. hemolyticus,* etc.), *Streptococcus pyogenes , Streptococcus agalactiae,* Streptococcal groups C-F (minute-colony streptococci), viridans streptococci, *Corynebacterium minutissimum, Clostridium* spp., or *Bartonella henselae;* uncomplicated acute urinary tract infections related to infection by *Staphylococcus aureus,* coagulase-negative staphylococcal species, or *Enterococcus* spp.; urethritis and cervicitis; sexually transmitted diseases related to infection by *Chlamydia trachomatis, Haemophilus ducreyi, Treponema pallidum, Ureaplasma urealyticum,* or *Neiserria gonorrheae;* toxin diseases related to infection by *S. aureus* (food poisoning and toxic shock syndrome), or Groups A, B, and C streptococci; ulcers related to infection by *Helicobacter pylori;* systemic febrile syndromes related to infection by *Borrelia recurrentis;* Lyme disease related to infection by *Borrelia burgdorferi;* conjunctivitis, keratitis, and dacrocystitis related to infection by *Chlamydia trachomatis, Neisseria gonorrhoeae, S. aureus, S. pneumoniae, S. pyogenes, H. influenzae,* or *Listeria* spp.; disseminated *Mycobacterium avium* complex (MAC) disease related to infection by *Mycobacterium avium,* or *Mycobacterium intracellulare;* infections caused by *Mycobacterium tuberculosis, M. leprae, M. paratuberculosis, M. kansasii,* or *M. chelonei;* gastroenteritis related to infection by *Campylobacter jejuni;* intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis or cardiovascular disease related to infection by *Helicobacter pylori* or *Chlamydia pneumoniae.* Bacterial infections and protozoal infections, and disorders related to such infections, which may be treated or prevented in animals include the following: bovine respiratory disease related to infection by *P. haemolytica, P. multocida, Mycoplasma bovis,* or *Bordetella* spp.; cow enteric disease related to infection by *E. coli* or protozoa (i.e., coccidia, cryptosporidia, etc.); dairy cow mastitis related to infection by *S. aureus, Strep. uberis, Streptococcus agalactiae, Streptococcus dysgalactiae, Klebsiella* spp., *Corynebacterium,* or *Enterococcus* spp.; swine respiratory disease related to infection by *A. pleuro., P. multocida,* or *Mycoplasma* spp.; swine enteric disease related to infection by *E. coli, Lawsonia intracellularis, Salmonella,* or *Serpulina hyodysinteriae;* cow footrot related to infection by *Fusobacterium* spp.; cow metritis related to infection by *E. coli;* cow hairy warts related to infection by *Fusobacterium necrophorum* or *Bacteroides nodosus*; cow pink-eye related to infection by *Moraxella bovis;* cow premature abortion related to infection by protozoa (i.e. neosporium); urinary tract infection in dogs and cats related to infection by *E. coli;* skin and soft tissue infections in dogs and cats related to infection by *S. epidermidis, S. intermedius,* coagulase neg. *Staphylococcus* or *P. multocida;* and dental or mouth infections in dogs and cats related to infection by *Alcaligenes* spp., *Bacteroides* spp., *Clostridium* spp., *Enterobacter* spp., *Eubacterium, Peptostreptococcus, Porphyromonas,* or *Prevotella.* Other bacterial infections and protozoal infections, and disorders related to such infections, which may be treated or prevented in accord with the method of the present invention are referred to in J. P. Sanford et al., "The Sanford Guide To Antimicrobial Therapy," 26th Edition, (Antimicrobial Therapy, Inc., 1996).

The term "halo", as used herein, unless otherwise indicated, includes fluoro, chloro, bromo or iodo. Preferred halo groups are fluoro, chloro and bromo.

The term "alkyl", as used herein, unless otherwise indicated, includes saturated monovalent hydrocarbon radicals having straight or branched moieties. Said alkyl group may include one or two double or triple bonds. It is understood that for said alkyl group to include a carbon-carbon double or triple bond at least two carbon atoms are required in said alkyl group.

The term "aryl", as used herein, unless otherwise indicated, includes an organic radical derived from an aromatic hydrocarbon by removal of one hydrogen, such as phenyl or naphthyl.

The term "4 to 10 membered heterocyclic", as used herein, unless otherwise indicated, includes aromatic and non-aromatic heterocyclic groups containing one or more heteroatoms each selected from O, S and N, wherein each heterocyclic group has from 4 to 10 atoms in its ring system. Non-aromatic heterocyclic groups include groups having only 4 atoms in their ring system, but aromatic heterocyclic groups must have at least 5 atoms in their ring system. The heterocyclic groups include benzo-fused ring systems and ring systems substituted with one or more oxo moieties. An example of a 4 membered heterocyclic group is azetidinyl (derived from azetidine). An example of a 5 membered heterocyclic group is thiazolyl and an example of a 10 membered heterocyclic group is quinolinyl. Examples of non-aromatic heterocyclic groups are pyrrolidinyl, tetrahydrofuranyl, tetrahydrothienyl, tetrahydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the compounds listed above, may be C-attached or N-attached where such is possible. For instance, a group derived from pyrrole may be pyrrol-1-yl (N-attached) or pyrrol-3-yl (C-attached).

The term "protecting group" refers to a suitable chemical group that may be attached to a functional group and removed at a later stage to reveal the intact functional group. Examples of suitable protecting groups for various functional groups are described in T.W. Greene and P.G.M. Wuts, Protective Groups in Organic Synthesis, 2d Ed., John Wiley and Sons (1991) (hereinafter, "Greene and Wuts"); L. Fieser and M. Fieser, Fieser and Fieser's Reagents for Organic Synthesis, John Wiley and Sons (1994); and L. Paquette, ed. Encyclopedia of Reagents for Organic Synthesis, John Wiley and Sons (1995).

The term "therapeutically effective amount" refers to an amount effective in treating or ameliorating disorders involving gastrointestinal motility or an amount effective in enhancing gastrointestinal motility in a patient, either as monotherapy or in combination with other agents. The term "treating" as used herein refers to the alleviation of symptoms of a particular disorder involving gastrointestinal motility in a patient, the improvement of an ascertainable measurement associated with a particular disorder, or the enhancement of gastrointestinal motility, e.g., to facilitate the placement of diagnostic or therapeutic instruments. As used herein, the term "patient" refers to mammals (including humans), fish and birds suffering from disorders characterized by impaired gastrointestinal motility or in need of enhanced gastrointestinal motility.

The term "pharmaceutically acceptable carrier" refers to a carrier that may be administered to a patient together with a compound of this invention. The carrier does not destroy the pharmacological activity of the prokinetic agent and is nontoxic when administered in doses sufficient to deliver a therapeutic amount of the prokinetic agent.

The phrase "pharmaceutically acceptable salt(s)", as used herein, unless otherwise indicated, includes salts of acidic or basic groups which may be present in the compounds of the present invention. The compounds of the present invention that are basic in nature are capable of forming a wide variety of salts with various inorganic and organic acids. The acids that may be used to prepare pharmaceutically acceptable acid addition salts of such basic compounds of are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds of the present invention that include a basic moiety, such as an amino group, may form pharmaceutically acceptable salts with various amino acids, in addition to the acids mentioned above.

As used herein, unless otherwise indicated, "Ac" indicates an acetyl group.

As used herein, unless otherwise indicated, "Me" indicates a methyl group.

As used herein, unless otherwise indicated, "Et" indicates an ethyl group.

Those compounds of the present invention that are acidic in nature are capable of forming base salts with various pharmacologically acceptable cations. Examples of such salts include the alkali metal or alkaline earth metal salts and, particularly, the calcium, magnesium, sodium and potassium salts of the compounds of the present invention.

The subject invention also includes isotopically-labeled compounds, and the pharmaceutically acceptable salts thereof, which are identical to those recited in formulas 1-7, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labeled compounds of formulas 1-7 of this invention and prodrugs thereof can generally be prepared by carrying out the procedures disclosed in the Schemes and/or in the Examples and Preparations below, by substituting a readily available isotopically labeled reagent for a non-isotopically labeled reagent.

This invention also encompasses pharmaceutical compositions containing and methods of treating bacterial infections through administering prodrugs of compounds of the formulas 1-7. Compounds of formulas 1-7 having free amino, amido, hydroxyl or carboxylic groups can be converted into prodrugs. Prodrugs include compounds wherein an amino acid residue, or a polypeptide chain of two or more (e.g., two, three or four) amino acid residues is covalently joined through an amide or ester bond to a free amino, hydroxyl or carboxylic acid group of compounds of formulas 1-7. The amino acid residues include but are not limited to the 20 naturally occurring amino acids commonly designated by three letter symbols and also includes 4-hydroxyproline, hydroxylysine, demosine, isodemosine, 3-methylhistidine, norvalin, beta-alanine, gamma-aminobutyric acid, citrulline homocysteine, homoserine, ornithine and methionine sulfone.

Additional types of prodrugs are also encompassed. For instance, free carboxyl groups can be derivatized as amides or alkyl esters. The amide and ester moieties may incorporate groups including but not limited to ether, amine and carboxylic acid functionalities. Free hydroxyl groups may be derivatized using groups including but not limited to hemisuccinates, phosphate esters, dimethylaminoacetates, and phosphoryloxymethyl-oxycarbonyls, as outlined in D. Fleisher, R. Bong, B.H. Stewart, Advanced Drug Delivery Reviews (1996) 19, 115. Carbamate prodrugs of hydroxyl and amino groups are also included, as are carbonate prodrugs and sulfate esters or phosphate esters of hydroxyl groups. Derivatization of hydroxyl groups as (acyloxy)methyl and (acyloxy)ethyl ethers wherein the acyl group may be an alkyl ester, optionally substituted with groups including but not limited to ether, amine and carboxylic acid functionalities, or where the acyl group is an amino acid ester as described above, are also encompassed. Prodrugs of this type are described in R.P. Robinson et al., J. Medicinal Chemistry (1996) 39, 10.

The present invention also includes pharmaceutical compositions for enhancing the motility of the gastrointestinal tract comprising a therapeutically effective amount of a compound of the invention and a pharmaceutically acceptable carrier or diluent. These pharmaceutical compositions may comprise one or more additional agents having a prokinetic effect or other therapeutic or prophylactic effect.

The present invention also includes a method of enhancing gastrointestinal motility in humans, other mammals, fish and birds in need of such treatment. This method can be used to treat disorders characterized by impaired gastrointestinal motility such as gastro-esophageal reflux disease, diabetic gastroparesis, delayed gastric emptying, pediatric gastroparesis, postoperative paralytic ileus, intestinal pseudoobstruction, gall bladder stasis, systemic sclerosis, anorexia, gastritis, emesis and chronic constipation. Alternatively, this method can be used to facilitate the placement of diagnostic and therapeutic instruments, such as the insertion of enteral feeding tubes into the proximal small intestine. The methods of the present invention comprise administering to a human, other mammal, fish or bird in need of such treatment a therapeutically effective amount of a compound of the invention, a pharmaceutically acceptable salt thereof or a pharmaceutical composition comprising the compound as defined above. This invention contemplates treatment methods in which the compounds of the present invention are administered either as a single agent or in combination with other therapeutic agents.

Unless otherwise indicated, references herein to gastrointestinal disorders include disorders that occur in mammals, fish and birds, as well as related disorders that may be treated or prevented by administering the compounds of the present invention.

Certain compounds of formulas 1-7 may contain one or more asymmetric carbons and may therefore exist in different isomeric forms. This invention includes all pure individual enantiomers and individual diastereomers of the compounds of formulas 1-7 and mixtures comprising any combination of these isomers. Each strereogenic carbon may be of the R or S configuration. In particular, the invention includes both the R and S configurations of the methyl group at C-8 and C-10 of the macrolide ring of formulas 1-7, and both the E and Z isomers of the - OR⁶ group connected to the nitrogen of the oxime moiety at C-9 of the macrolide ring of formulas 1-7. Although specific compounds exemplified in this application may be depicted in a particular stereochemical configuration, compounds having either the opposite stereochemistry at any given chiral center or mixtures thereof are also envisioned. The compounds of formulas 1-7 may additionally exist as tautomers. This invention includes all such pure tautomers and mixtures thereof. The invention includes uses of any of the above compounds or mixtures of the compounds.

### Detailed Description of the Invention

The following reaction schemes illustrate the preparation of the compounds of the present invention. Unless otherwise indicated a, R¹, R², R³, R⁴, R⁵, R⁶, R^{7a}, R¹², W, X¹, X², Y and Z are defined as above.

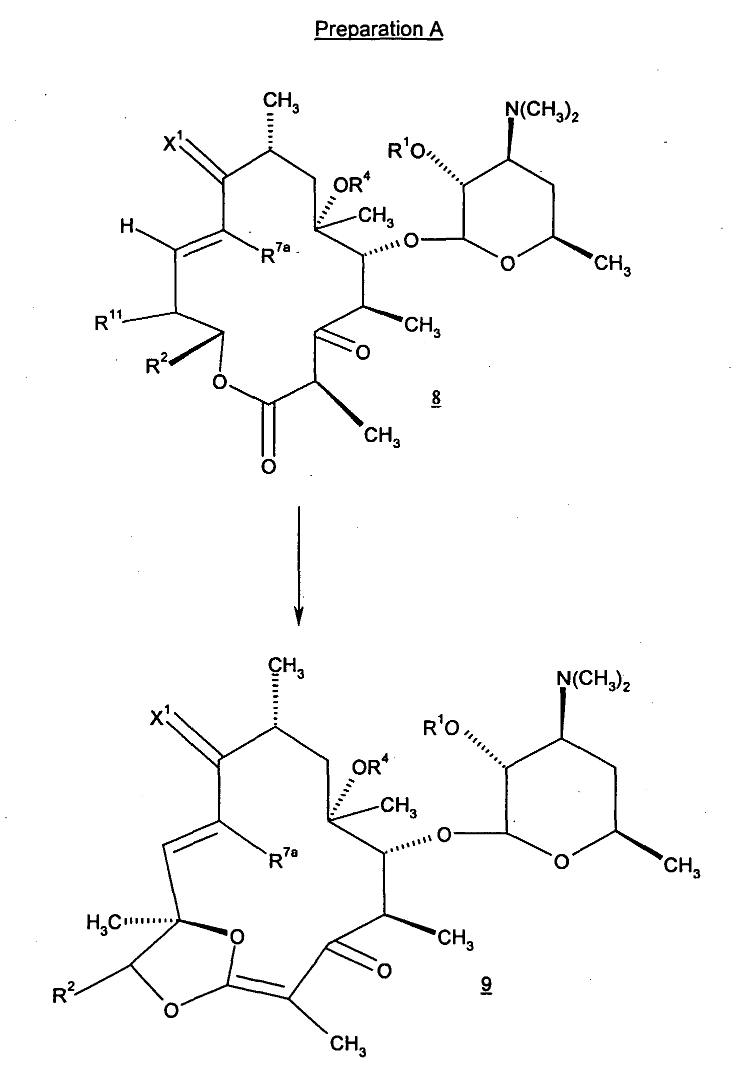

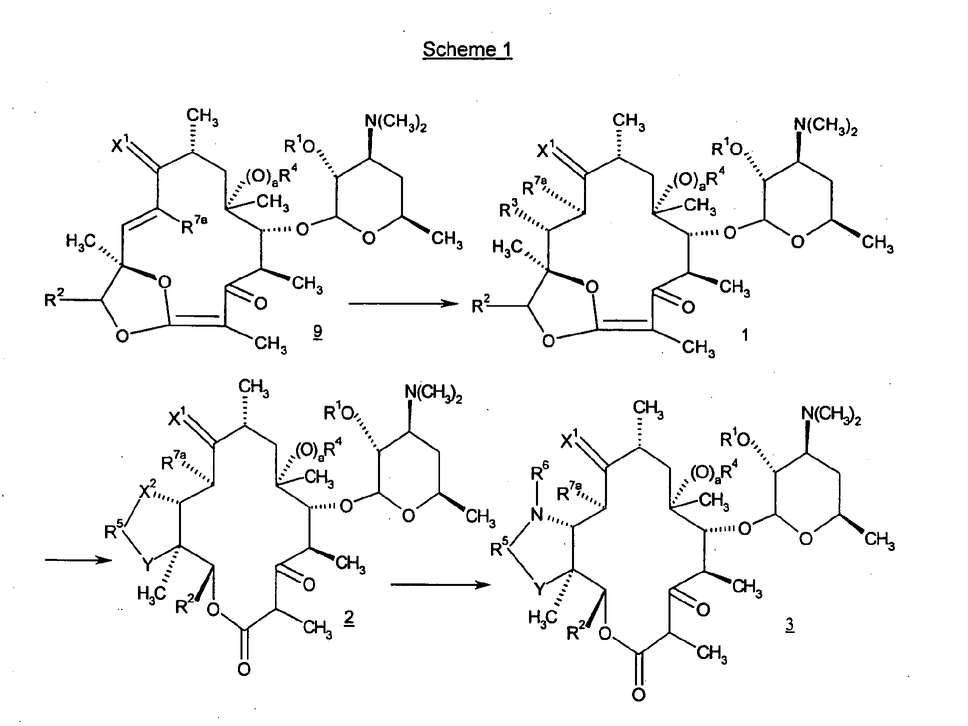

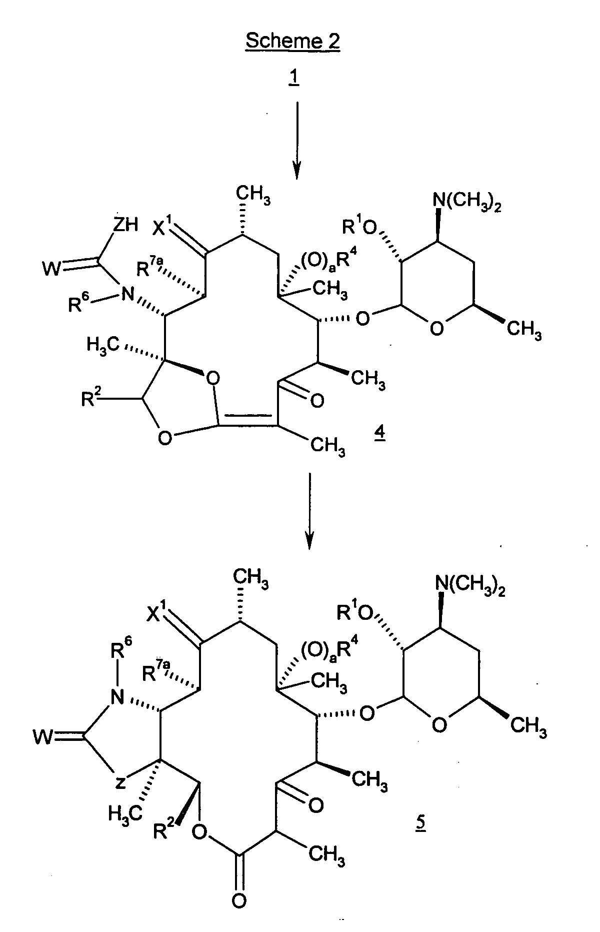

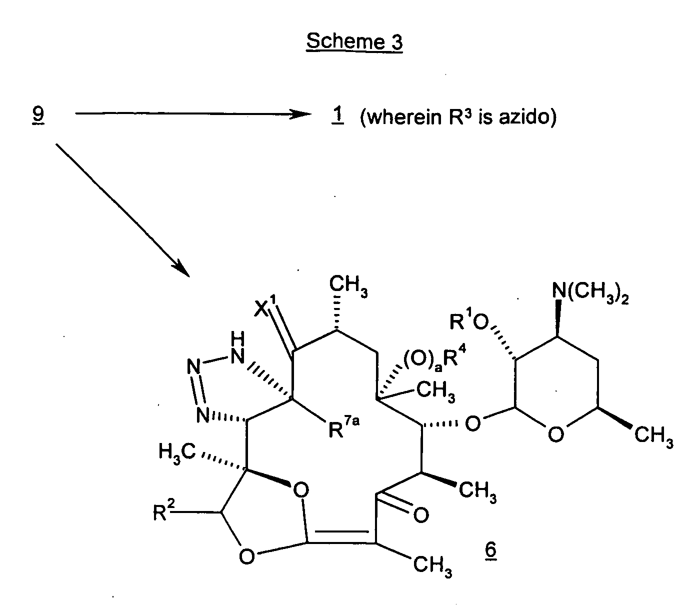

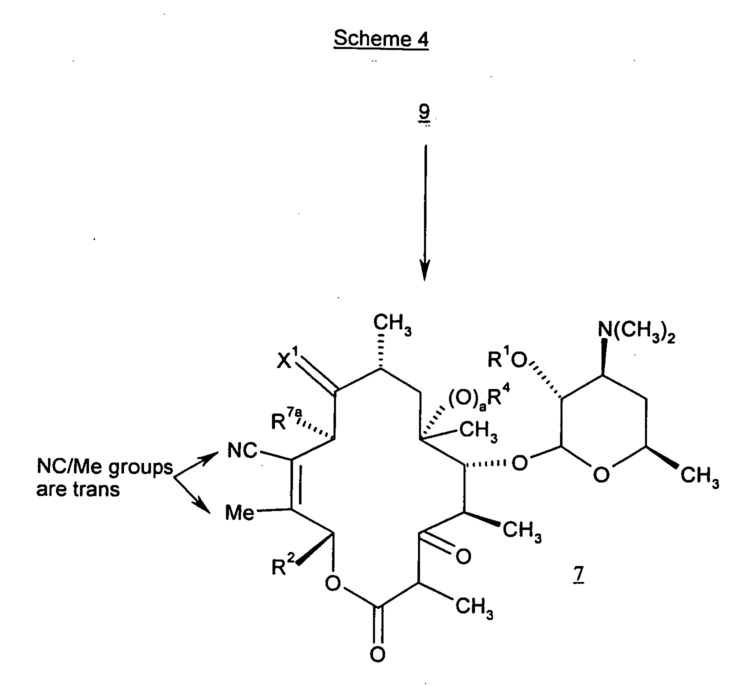

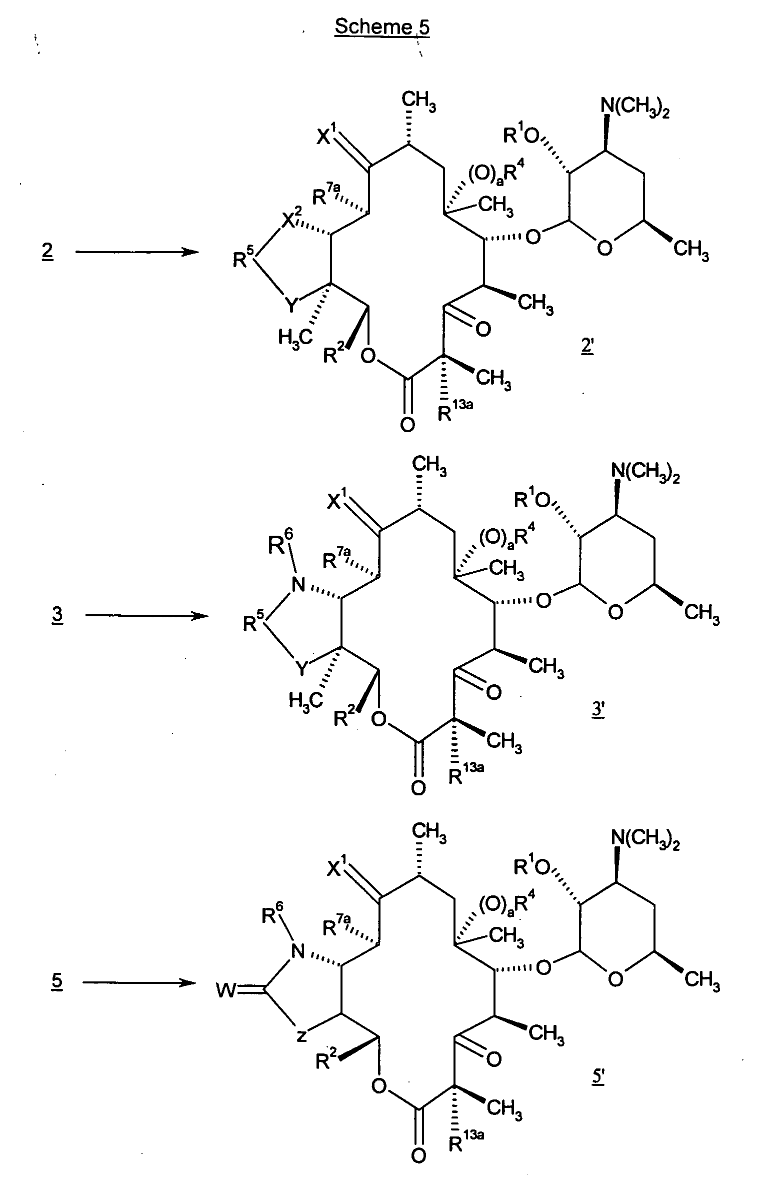

In the reaction of Preparation A, the compound of formula 8 wherein R¹¹ is a leaving group, such as (C₁-C₆)alkylsulfonyloxy, (C₆-C₁₀)arylsulfonyloxy, (C₁-C₆)acyloxy or imidizolyl-carbonyloxy, is converted to the corresponding ketene acetal compound of formula 9 by treating 8 with a base, such as 1,8-diazabicyclo[5.4.0]undec-7-ene, 1,5-diazabicyclo[4.3.0]non-5-ene, ethyldiisopropylamine, triethylamine, lithium hexamethyldisilazide or potassium hexamethyldisilazide, preferably 1,8-diazabicyclo[5.4.0]unded-7ene, in the presence of a polar aprotic solvent, such as acetonitrile, dimethylformamide, tetrahydrofuran, preferably acetonitrile. The reaction is stirred at a temperature between about 20°C to about 100°C, preferably about 80°C, for a time period between about 0.5 hours to about 6 hours, preferably about 2 hours. The starting compound of formula 8 can be prepared as described in United States Patent 5,543,400. The starting compounds of formula 8 wherein R⁴ is various groups can be prepared as described in WO 98/09978.

In the reaction of Scheme 1, conversion of 9 to 1 is affected by mixing ketene acetal 9 with a nucleophile at room temperature or elevated temperature (up to 100°C) in an inert solvent such as acetonitrile or trifluoroethanol. The process can be catalyzed by addition of acid such as acetic acid or base such as triethylamine.

When R³ is R⁶NH-, R⁶S-, or R⁶O- and R⁶ is substituted with a terminal amino, hydroxyl or thiol group, or a protected amino, protected hydroxyl, or protected thiol group, compound 1 can then be converted to 2 by heating this compound in an inert solvent such as acetonitrile or THF. The temperature can be between room temperature to 100°C. Some salts such as Yb(OTf)₃ and Sc(OTf)₃ can be used as a catalyst. The process also can be catalyzed by addition of acid such as acetic acid or SnCl₄.

When X² is NH, compound 2 can be further converted to 3 by a reductive amination with R⁶-CHO and a reducing agent such as NaBH₃CN or NaBH(OAc)₃. This reaction can be carried out at 0° to 50°C in a solvent such as ethanol, acetic acid, or THF.

In Scheme 2, compound 1, in which R³ is R⁶NH, is first converted to 4 by the action of phosgene or carbonyl dimidazole and ammonia or amine in an inert solvent such as THF. Compound 4 can then be converted to 5 by heating to 50° to 100°C in an inert solvent such as acetonitrile or THF. The process also can be catalyzed by addition of acid such as acetic acid or SnCl₄. Alternatively, compound 4 can be converted to 5 by the action of SnCl₄ and NBS in an aprotic solvent such as CH₂Cl₂ at low temperatures, preferably between -20° to 0°C.

In Scheme 3, compound 9 is converted to 1 and 6 by the action of azidotrimethylsilane and tetrabutylammonium fluoride in an inert solvent such as THF at room temperature wherein R³ of formula 1 is N₃.

In Scheme 4, compound 9 is converted to 7 by the action of trimethylsilyl cyanide and tetrabutylammonium fluoride in an inert solvent such as THF at room temperature.

In Scheme 5, when R¹ is a protecting hydroxyl group, compound 2, 3 or 5 generated in Scheme 1 or 2 can be halogenated at C2 and converted to compound 2', 3' or 5'. Compound 2, 3 or 5 is treated with a strong base such as KHMDS (potassium hexamethyldisilazide) at low temperature (-78°C) in CH₂Cl₂ and then treated with a halogenating agent such as SELECTFLUOR™. R^{13a} in compound 2', 3' or 5' can be Fl, Cl, Br or I.

The compounds of the present invention may have newly generated asymmetric carbon atoms. Such diastereomeric mixtures can be separated into their individual diastereomers on the basis of their physical chemical differences by methods known to those skilled in the art, for example, by chromatography or fractional crystallization. All such isomers, including diastereomeric mixtures and pure enantiomers are considered as part of the invention. The C₈ epimers are specifically claimed.

The compounds of formulas 1-7 that are basic in nature are capable of forming a wide variety of different salts with various inorganic and organic acids. Although such salts must be pharmaceutically acceptable for administration to animals, it is often desirable in practice to initially isolate the compound of formulas 1-7 from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

Those compounds of formula 1-7 that are acidic in nature are capable of forming base salts with various pharmacologically acceptable cations. Examples of such salts include the alkali metal or alkaline-earth metal salts and particularly, the sodium and potassium salts. These salts may be prepared by conventional techniques. The chemical bases which are used as reagents to prepare the pharmaceutically acceptable base salts of this invention are those which form non-toxic base salts with the acidic compounds of formulas 1-7. Such non-toxic base salts include those derived from such pharmacologically acceptable cations as sodium, potassium calcium and magnesium, etc. These salts can be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The activity of the compounds of the present invention against bacterial and protozoa pathogens is demonstrated by the compound's ability to inhibit growth of defined strains of human (Assay I) or animal (Assays II and III) pathogens.

### Assay I

Assay I, described below, employs conventional methodology and interpretation criteria and is designed to provide direction for chemical modifications that may lead to compounds that circumvent defined mechanisms of macrolide resistance. In Assay I, a panel of bacterial strains is assembled to include a variety of target pathogenic species, including representatives of macrolide resistance mechanisms that have been characterized. Use of this panel enables the chemical structure/activity relationship to be determined with respect to potency, spectrum of activity, and structural elements or modifications that may be necessary to obviate resistance mechanisms. Bacterial pathogens that comprise the screening panel are shown in the table below. In many cases, both the macrolide-susceptible parent strain and the macrolide-resistant strain derived from it are available to provide a more accurate assessment of the compound's ability to circumvent the resistance mechanism. Strains that contain the gene with the designation of *ermA*/*ermB*/*ermC* are resistant to macrolides, lincosamides, and streptogramin B antibiotics due to modifications (methylation) of 23S rRNA molecules by an Erm methylase, thereby generally prevent the binding of all three structural classes. Two types of macrolide efflux have been described; *msrA* encodes a component of an efflux system in staphylococci that prevents the entry of macrolides and streptogramins while *mefA*/*E* encodes a transmembrane protein that appears to efflux only macrolides. Inactivation of macrolide antibiotics can occur and can be mediated by either a phosphorylation of the 2'-hydroxyl *(mph)* or by cleavage of the macrocyclic lactone (esterase). The strains may be characterized using conventional polymerase chain reaction (PCR) technology and/or by sequencing the resistance determinant. The use of PCR technology in this application is described in J. Sutcliffe et al., "Detection Of Erythromycin-Resistant Determinants By PCR", Antimicrobial Agents and Chemotherapy, 40(11), 2562-2566 (1996). The assay is performed in microtiter trays and interpreted according to Performance Standards for Antimicrobial Disk Susceptibility Tests - Sixth Edition: Approved Standard, published by The National Committee for Clinical Laboratory Standards (NCCLS) guidelines; the minimum inhibitory concentration (MIC) is used to compare strains. Compounds are initially dissolved in dimethylsulfoxide (DMSO) as 40 mg/ml stock solutions.

| Strain Designation | Macrolide Resistance Mechanism(s) |
|---|---|
| Staphylococcus aureus 1116 | susceptible parent |
| Staphylococcus aureus 1117 | ermB |
| Staphylococcus aureus 0052 | susceptible parent |
| Staphylococcus aureus 1120 | ermC |
| Staphylococcus aureus 1032 | msrA, mph, esterase |
| Staphylococcus hemolyticus 1006 | msrA, mph |
| Streptococcus pyogenes 0203 | susceptible parent |
| Streptococcus pyogenes 1079 | ermB |
| Streptococcus pyogenes 1062 | susceptible parent |
| Streptococcus pyogenes 1061 | ermB |
| Streptococcus pyogenes 1064 | ermB |
| Streptococcus agalactiae 1024 | susceptible parent |
| Streptococcus agalactiae 1023 | ermB |
| Streptococcus pneumoniae 1016 | susceptible |
| Streptococcus pneumoniae 1046 | ermB |
| Streptococcus pneumoniae 1095 | ermB |
| Streptococcus pneumoniae 1175 | mefE |
| Streptococcus pneumoniae 0085 | susceptible |
| Haemophilus influenzae 0131 | susceptible |
| Moraxella catarrhalis 0040 | susceptible |
| Moraxella catarrhalis 1055 | erythromycin intermediate resistance |
| Escherichia coli 0266 | susceptible |

Assay II is utilized to test for activity against *Pasteurella multocida* and Assay III is utilized to test for activity against *Pasteurella haemolytica.*

### Assay II

This assay is based on the liquid dilution method in microliter format. A single colony of *P. multocida* (strain 59A067) is inoculated into 5 ml of brain heart infusion (BHI) broth. The test compounds are prepared by solubilizing 1 mg of the compound in 125 µl of dimethylsulfoxide (DMSO). Dilutions of the test compound are prepared using uninoculated BHI broth. The concentrations of the test compound used range from 200 µg/ml to 0.098 µg/ml by two-fold serial dilutions. The *P. multocida* inoculated BHI is diluted with uninoculated BHI broth to make a 10⁴ cell suspension per 200 µl. The BHI cell suspensions are mixed with respective serial dilutions of the test compound, and incubated at 37°C for 18 hours. The minimum inhibitory concentration (MIC) is equal to the concentration of the compound exhibiting 100% inhibition of growth of P. multocida as determined by comparison with an uninoculated control.

### Assay III

This assay is based on the agar dilution method using a Steers Replicator. Two to five colonies isolated from an agar plate are inoculated into BHI broth and incubated overnight at 37°C with shaking (200 rpm). The next morning, 300 µl of the fully grown *P. haemolytica* preculture is inoculated into 3 ml of fresh BHI broth and is incubated at 37°C with shaking (200 rpm). The appropriate amounts of the test compounds are dissolved in ethanol and a series of two-fold serial dilutions are prepared. Two ml of the respective serial dilution is mixed with 18 ml of molten BHI agar and solidified. When the inoculated *P. haemolytica* culture reaches 0.5 McFarland standard density, about 5 µl of the *P. haemolytica* culture is inoculated onto BHI agar plates containing the various concentrations of the test compound using a Steers Replicator and incubated for 18 hours at 37°C. Initial concentrations of the test compound range from 100-200 µg/ml. The MIC is equal to the concentration of the test compound exhibiting 100% inhibition of growth of *P. haemolytica* as determined by comparison with an uninoculated control.

The in vivo activity of the compounds of formulas 1-7 can be determined by conventional animal protection studies well known to those skilled in the art, usually carried out in mice.

Mice are allotted to cages (10 per cage) upon their arrival, and allowed to acclimate for a minimum of 48 hours before being used. Animals are inoculated with 0.5 ml of a 3 x 10³ CFU/ml bacterial suspension *(P. multocida* strain 59A006) intraperitoneally. Each experiment has at least 3 non-medicated control groups including one infected with 0.1X challenge dose and two infected with 1X challenge dose; a 10X challenge data group may also be used. Generally, all mice in a given study can be challenged within 30-90 minutes, especially if a repeating syringe (such as a Cornwall® syringe) is used to administer the challenge. Thirty minutes after challenging has begun, the first compound treatment is given. It may be necessary for a second person to begin compound dosing if all of the animals have not been challenged at the end of 30 minutes. The routes of administration are subcutaneous or oral doses. Subcutaneous doses are administered into the loose skin in the back of the neck whereas oral doses are given by means of a feeding needle. In both cases, a volume of 0.2 ml is used per mouse. Compounds are administered 30 minutes, 4 hours, and 24 hours after challenge. A control compound of known efficacy administered by the same route is included in each test. Animals are observed daily, and the number of survivors in each group is recorded. The *P. multocida* model monitoring continues for 96 hours (four days) post challenge.

The PD₅₀ is a calculated dose at which the compound tested protects 50% of a group of mice from mortality due to the bacterial infection which would be lethal in the absence of drug treatment.

The compounds of formulas 2, 3, and 5 and the pharmaceutically acceptable salts, prodrugs and solvates thereof (hereinafter "the active compounds"), may be administered through oral, parenteral, topical, or rectal routes in the treatment or prevention of bacterial or protozoa infections. In general, these compounds are most desirably administered in dosages ranging from about 0.2 mg per kg body weight per day (mg/kg/day) to about 200 mg/kg/day in single or divided doses (i.e., from 1 to 4 doses per day), although variations will necessarily occur depending upon the species, weight and condition of the subject being treated and the particular route of administration chosen. However, a dosage level that is in the range of about 4 mg/kg/day to about 50 mg/kg/day is most desirably employed. Variations may nevertheless occur depending upon the species of mammal, fish or bird being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds may be administered alone or in combination with pharmaceutically acceptable carriers or diluents by the routes previously indicated, and such administration may be carried out in single or multiple doses. More particularly, the active compounds may be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active compound may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of an active compound in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques will known to those skilled in the art.

Additionally, it is also possible to administer the active compounds of the present invention topically and this may be done by way of creams, jellies, gels, pastes, patches, ointments and the like, in accordance with standard pharmaceutical practice.

For administration to animals other than humans, such as cattle or domestic animals, the active compounds may be administered in the feed of the animals or orally as a drench composition.

The active compounds may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active compounds may also be coupled with soluble polymers as targetable drug carriers. Such polymers can include polyvinylpyrrolidone, pyran copolymer, polyhydroxypropylmethacrylamide phenyl, polyhydroxyethylaspartamide-phenol, or polyethyleneoxide-polylysine substituted with palmitoylresidues. Furthermore, the active compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

The compounds of this invention may be co-administered with other compounds of this invention or with other prokinetic compounds to increase the effect of therapy. Combination therapies according to this invention may exert an additive or synergistic prokinetic effect, e.g., because each component agent of the combination may act on a different site or through a different mechanism. The use of such combination therapies may also advantageously reduce the dosage of a given conventional prokinetic agent that would be required for a desired therapeutic effect, as compared to when that agent is administered as a monotherapy. Such combinations may reduce or eliminate the side effects of conventional prokinetic therapies, while not interfering with the prokinetic activity of those agents. These combinations reduce the potential of resistance to single agent therapies, while minimizing any associated toxicity. Alternatively, pharmaceutical compositions according to this invention may be comprised of a combination of a compound of this invention and another agent having a different therapeutic or prophylactic effect.

The Examples provided below illustrate specific embodiments of the invention, but the invention is not limited in scope to the Examples specifically exemplified.

### Example 1

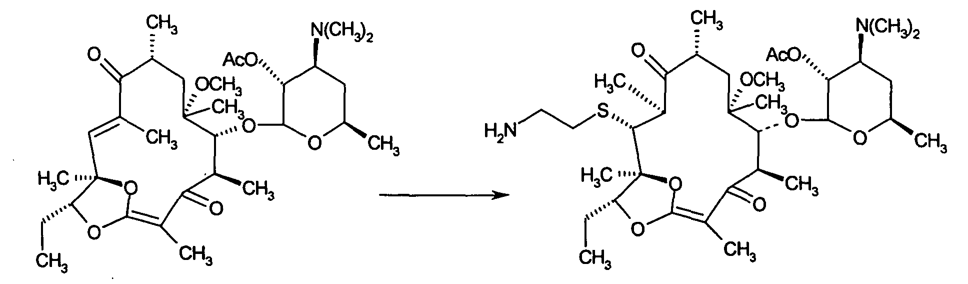

To a solution of ketene acetal (9, 6 mg, 0.01 mmol) in 5 mL of ethanol was added 2-aminoethone thiol (1 mg, 0.01 mmol). After stirring 1 hour at room temperature, ethanol was removed under reduced pressure. The residue was dissolved in ethyl acetate and washed with 0.1 M sodium hydroxide solution and brine. It was dried over Na₂SO₄ and evaporated. The residue was chromatographed on SiO₂ (TLC plate, 6% MeOH-0.5% NH₄OH-CH₂ Cl₂) to give 2 mg of the purified product: MS m/e 671 (M+I).

### Example 2

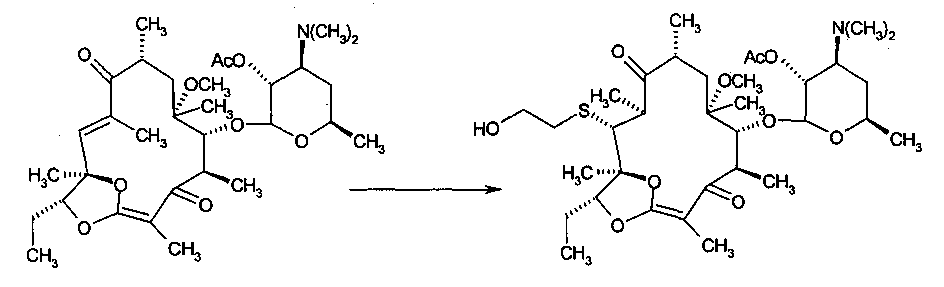

Ketene acetal (9, 100 mg, 0.17 mmol) was dissolved in 1 mL of 2-mercaptoethanol and stirred at room temperature for 1 hour. The reaction mixture was diluted with ethyl acetate and washed with 0.05 M sodium hydroxide solution. The organic layer was further washed with water and brine, and dried over Na₂SO₄. Ethyl acetate was removed under reduced pressure and the residue was chromatographed on SiO₂ (preparative TLC, acetone:hexane = 1:1) to give 30 mg of the desired product: MS m/e 672 (M+I).

### Example 3

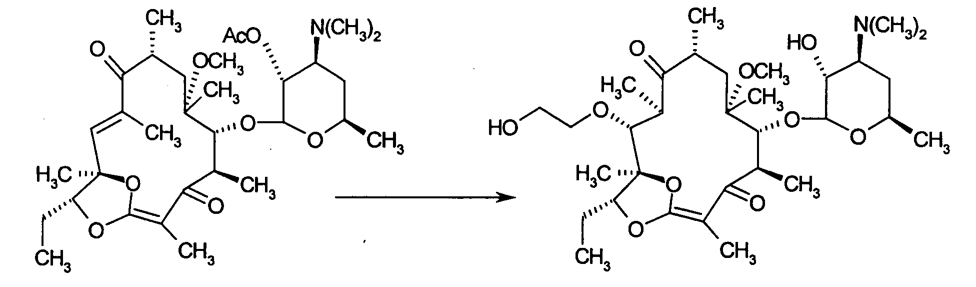

Ketene acetal (9, 28 mg, 0.03 mmol) was suspended in 2 mL of ethylene glycol. A small amount of acetonitrile was added to make a homogeneous solution. The solution was heated to 60°C for 4 hours. The reaction mixture was diluted with water and extracted three times with ethyl acetate. The ethyl acetate was washed with water and dried over Na₂SO₄. Evaporation of the solvent gave 28 mg of crude product: MS m/e 614 (M+I).

### Example 4

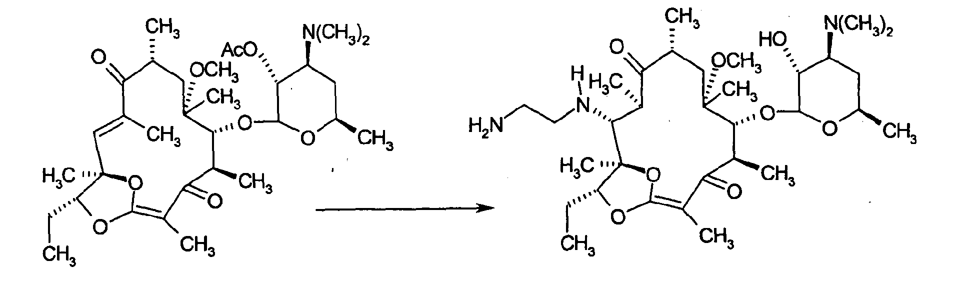

Ketene acetal (9, 28 mg, 0.05 mmol) was dissolved in 3mL of ethylenediamine and warmed to 60 C for 1 hour. The reaction mixture was poured into 20 mL of water. The resulting aqueous layer was extracted with ethyl acetate. The ethyl acetate layer was washed with water and brine. Drying over Na₂SO₄ and evaporation of the solvent gave 26 mg of crude product: MS m/e 653 (M+1).

### Example 5

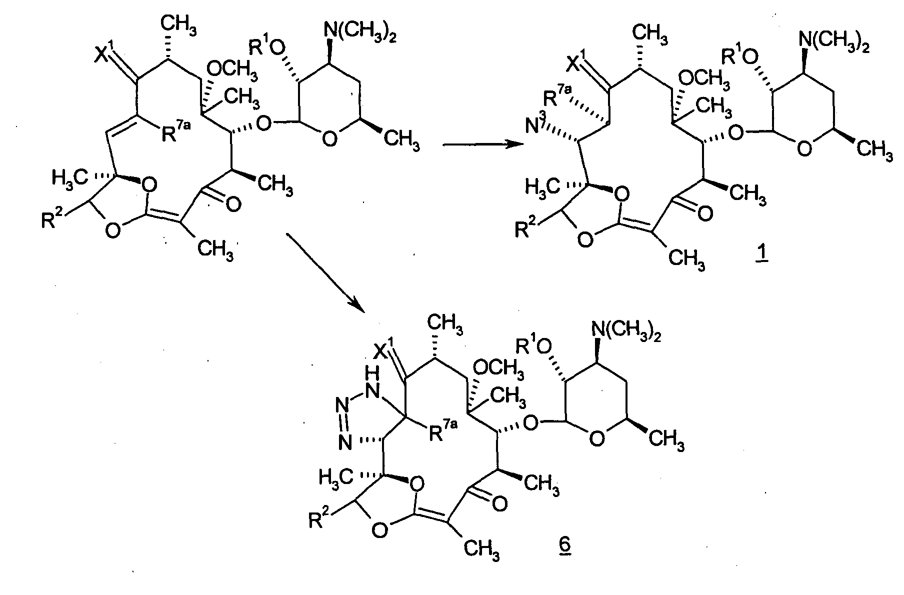

To a solution of azidotrimethylsilane (26µl, 0.20 mmol) and tetrabutylammonium fluoride (190 µl of 1 M solution in THF) in 3 mL of THF was added ketene acetal (9, 100 mg, 0.17 mmol). The solution was stirred at room temperature for 3 days. The solvent was evaporated and the residue was chromatographed on SiO₂ (prep. TLC, acetone:hexane = 1:1) to give 27 mg of 1 :MS m/e 637 (M+I); and 7 mg of 6: MS m/e 637 (M+I).

### Example 6

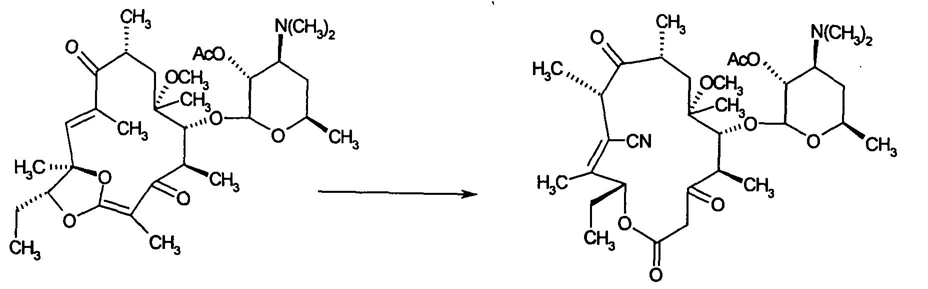

To a solution of trimethylsilyl cyanide (27µl, 0.20 mmol) and tetrabutylammonium fluoride (253 µl of 1 M solution in THF, 0.26 mmol) in 3mL of THF was added ketene acetal (9 150 mg, 0.17 mmol). The solution was stirred at room temperature for 1 hour, then diluted with ethyl acetate and washed with water and brine. The organic layer was dried over Na₂SO₄ and evaporated. The residue was chromatographed on SiO₂ (acetone:hexane = 3:7) to give 75 mg of 7; MS m/e 621 (M+I).

## Claims

1. A compound of the formula 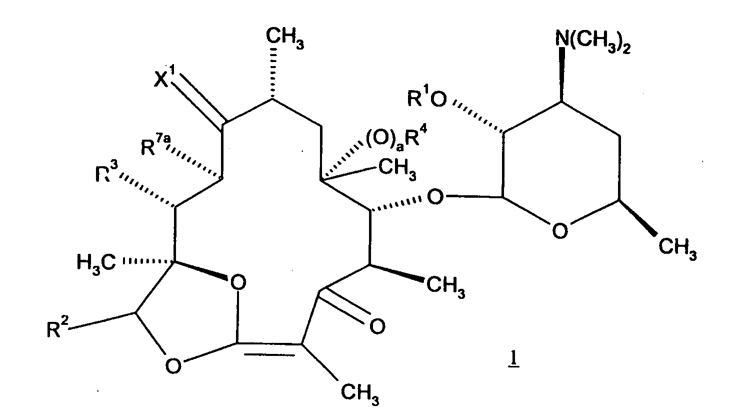 or 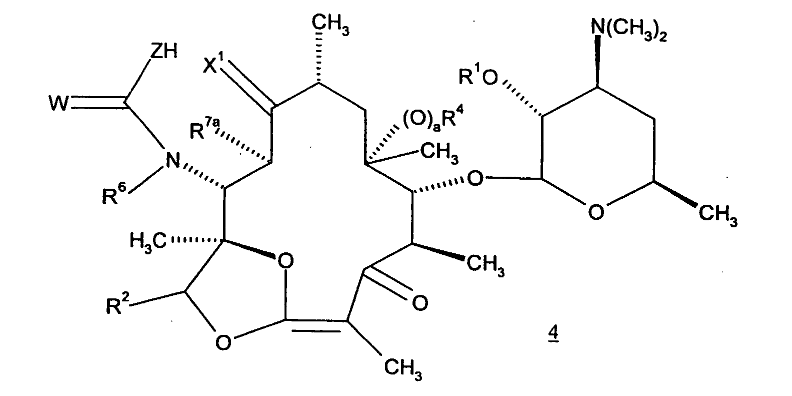 or 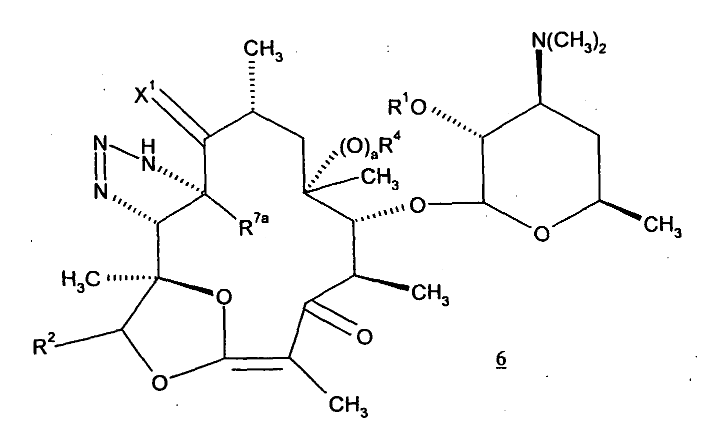 or 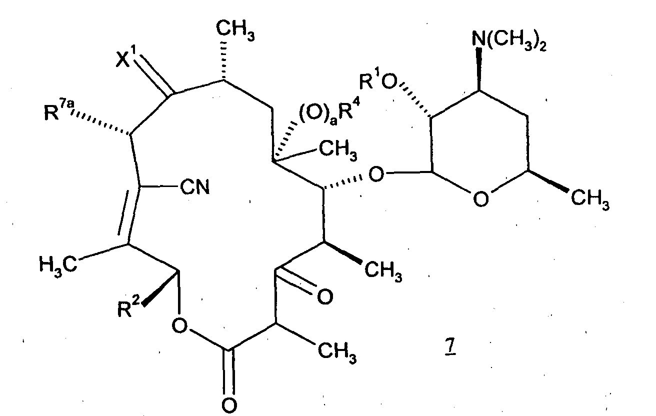 or a salt or solvate thereof, wherein:
a is 0 or 1;
X¹ is =O or =NOR⁶;
R¹ is hydrogen or a hydroxyl protecting group;
R² is hydrogen, C₁-C₆ alkyl, C₃-C₁₀ cycloalkyl, C₅-C₁₀ cycloalkenyl, C₂-C₆ alkenyl, C₂-C₆ alkynyl, (C₁-C₆ alkoxy)C₁-C₆ alkyl, (C₁-C₆ alkylthio)C₁-C₆ alkyl, -(CR¹⁰R¹¹)ₘ(C₆-C₁₀ aryl), and - (CR¹⁰R¹¹)ₘ(4 to 10 membered heterocyclic), wherein each m is independently an integer from 0 to 6, and the foregoing R² groups, except hydrogen, are optionally substituted by one to three R⁸ substituents;
R³ is hydrogen or methyl optionally substituted by one or two groups independently selected from nitro, cyano, R⁶C(O) and R⁶OC(O); or R³ is azido, -OR⁶, -(CR¹⁰R¹¹ₘNR¹⁰R⁶ or - SR⁶;
R⁴ is hydrogen or C₁-C₁₀ alkyl optionally substituted by one or two R⁸ groups;
each R⁶ is independently selected from hydrogen, C₁-C₆ alkyl, C₂-C₁₂ alkenyl, C₂-C₁₂ alkynyl, (C₃-C₁₀ cycloalkyl)C₁-C₆ alkyl, -(CR¹⁰R¹¹)ₘ(C₆-C₁₀ aryl), and -(CR¹⁰R¹¹)ₘ(4 to 10 membered heterocyclic), wherein each m is independently an integer from 0 to 6; and the foregoing R⁶ groups, except hydrogen, are optionally substituted by one to three R⁸ substituents;
R^{7a} is hydrogen or methyl;
each R⁸ is independently selected from C₁-C₁₀ alkyl, C₂-C₁₀ alkenyl, C₂-C₁₀ alkynyl, oxo, halo, cyano, nitro, trifluoromethyl, difluoromethoxy, trifluoromethoxy, azido, -OR⁹, -C(O)R⁹, - C(O)OR⁹, -NR¹⁰C(O)OR¹², -OC(O)R⁹, -NR¹⁰SO₂R¹², -SO₂NR⁹R¹⁰, -NR¹⁰C(O)R⁹, -C(O)NR⁹R¹⁰, -NR⁹R¹⁰, -S(O)ⱼ(CR¹⁰R¹¹)ₘ(C₆-C₁₀ aryl), -S(O)ⱼ(C₁-C₆ alkyl), wherein j is an integer from 0 to 2, - (CR¹⁰R¹¹)ₘ(C₆-C₁₀ aryl), -O(CR¹⁰R¹¹)ₘ(C₆-C₁₀ aryl), -NR¹⁰(CR¹⁰R¹¹)ₘ(C₆-C₁₀ aryl), and -(CR¹⁰R¹¹)ₘ(4 to 10 membered heterocyclic), wherein each m is independently an integer from 0 to 4; said alkyl, alkenyl and alkynyl groups optionally contain 1 or 2 hetero moieties selected from O, -S(O)ⱼ- wherein j is an integer from 0 to 2, and -N(R⁹)- with the proviso that two O atoms, two S atoms, or an O and S atom are not attached directly to each other, and the proviso that an O atom, a S atom or a N atom are not attached directly to a triple bond or a non-aromatic double bond; said cycloalkyl, aryl and heterocyclic R² groups are optionally fused to a C₆-C₁₀ aryl group, a C₅-C₈ cycloalkyl group, or a 4 to 10 membered heterocyclic group; and said alkyl, cycloalkyl, aryl and heterocyclic R² groups are optionally substituted by 1 to 5 substituents independently selected from oxo, halo, cyano, nitro, trifluoromethyl, difluoromethoxy, trifluoromethoxy, azido, -NR¹⁰SO₂R¹², -SO₂NR⁹R¹⁰, -C(O)R⁹, -C(O)OR⁹, -OC(O)R⁹, -NR¹⁰C(O)OR¹², -NR¹⁰C(O)R⁹, -C(O)NR⁹R¹⁰, -NR⁹R¹⁰, -OR⁹, C₁-C₁₀ alkyl, -(CR¹⁰R¹¹)ₘ(C₆-C₁₀ aryl), and -(CR¹⁰R¹¹)ₘ(4 to 10 membered heterocyclic), wherein each m is independently an integer ranging from 0 to 4;
each R⁹ is independently selected from H, C₁-C₁₀ alkyl, -(CR¹⁰R¹¹)ₘ(C₆-C₁₀ aryl), and -(CR¹⁰R¹¹)ₘ(4 to 10 membered heterocyclic), wherein each m is independently an integer from 0 to 4; said alkyl group optionally includes 1 or 2 hetero moieties selected from O, -S(O)ⱼ- wherein j is an integer ranging from 0 to 2, and -N(R¹⁰)- with the proviso that two O atoms, two S atoms, or an O and S atom are not attached directly to each other; said cycloalkyl, aryl and heterocyclic R⁹ groups are optionally fused to a C₆-C₁₀ aryl group, a C₅-C₈ cycloalkyl group, or a 4 to 10 membered heterocyclic group; and the foregoing R⁹ substituents, except H, are optionally substituted by 1 to 5 substituents independently selected from oxo, halo, cyano, nitro, trifluoromethyl, difluoromethoxy, trifluoromethoxy, azido, -C(O)R¹⁰, -C(O)OR¹⁰, -OC(O)R¹⁰, -NR¹⁰C(O)R¹¹, -C(O)NR¹⁰R¹¹, -NR¹⁰R¹¹, hydroxy, C₁-C₆ alkyl, and C₁-C₆ alkoxy, and with the proviso that R⁹ must be attached through a carbon atom unless R⁹ is H;
each R¹⁰ and R¹¹ is independently H or C₁-C₆ alkyl;
each R¹² is selected from the substituents provided in the definition of R⁹ except R¹² is not H;
W is O or S;
Z is -N(R⁶)-, O, S, or -CH(R¹⁴)- wherein R¹⁴ is H, NO₂, CN, -C(O)R⁹ or C(O)OR⁹; and with the proviso that when a is zero, R⁴ is hydrogen.

2. A compound according to claim 1 wherein said compound is a compound of formula 1 and a is 1, X¹ is O, R¹ is H, R² is CH₂CH₃ and R⁴ is H.

3. A compound according to claim 2 wherein R³ is S(CH₂)₂NH₂, S(CH₂)₂OH, O(CH₂)₂OH or NH(CH₂)₂OH.

4. A compound according to claim 1 wherein said compound is a compound of formula 7 and a is 1, X¹ is O, R¹ is H, R² is CH²CH³ and R⁴ is CH³.
